# EUROPEAN PATENT APPLICATION

(11) **EP 3 308 790 A1**
(43) Date of publication of application: **18.04.2018**
(21) Application number: 16807597.6
(22) Date of filing: 10.06.2016
(51) Int. Cl.: A61K 31/724, A61K 31/132, A61K 31/7016, A61K 31/7105, A61P 25/00, A61P 25/14, A61P 25/28, A61P 43/00

(54) **NOVEL AMYLOID FIBRIL FORMATION INHIBITOR**

(30) Priority: 10.06.2015 JP 2015117150
(71) Applicant: National University Corporation Kumamoto University, Kumamoto-shi Kumamoto 860-8555 (JP)
(72) Inventor: JONO, Hirofumi, Kumamoto-shi Kumamoto 860-8555 (JP); ARIMA, Hidetoshi, Kumamoto-shi Kumamoto 860-8555 (JP); ANDO, Yukio, Kumamoto-shi Kumamoto 860-8555 (JP); MOTOYAMA, Keiichi, Kumamoto-shi Kumamoto 860-8555 (JP); HIGASHI, Taishi, Kumamoto-shi Kumamoto 860-8555 (JP)
(74) Representative: Parchmann, Stefanie
(86) International application number: PCT/JP2016/067373
(87) International publication number: WO 2016/199892

(57) **Abstract**

The purpose of the present invention is to provide a therapeutic agent that is more effective in refractory amyloidosis. More specifically, it is to provide a novel substance that is highly safe and has a TTR protein amyloid fibril formation-inhibiting effect with which it is possible to inhibit the formation of amyloid fibrils better than conventional therapeutic agents. Provided by the invention is an amyloid fibril formation inhibitor containing as an active ingredient a complex comprsing a conjugate (GUG-B-CDE) of glucuronylglucosyl-B-cyclodextrin (GUG-B-CyD) and polyamide amine dendrimer having an alkylene diamine as the core and RNA that causes RNA interference in the mRNA of transthyretin (TTR). Also provided by the present invention is a pharmaceutical composition for the prevention and/or treatment of amyloidosis including the amyloid fibril formation inhibitor.

## Description

### Technical Field

The present invention relates to a novel amyloid fibril suppressant. Further, the present invention relates to a pharmaceutical composition comprising the suppressant for prevention and/or treatment of amyloidosis.

### Background Art

Amyloidosis denotes a syndrome in which proteins having a β-sheet structure are polymerized to form insoluble microfibril called amyloid or amyloid fibril, and the microfibril deposits in vivo to cause tissue injury. Virchow, the German pathologist, found that the tissue specimen of amyloidosis stains purple with iodine. Based on this result, Virchow supposed that the substance deposited to tissue is polysaccharide, named it "starch-like substance", namely "amyloid" and proposed that the pathological condition caused by deposition of amyloid is called amyloidosis.
In the subsequent studies, it was found that the main components of amyloid are proteins that polymerize in the form of nylon to form fibril, and the serum amyloid P component, glycosaminoglycan and the like are contained in amyloid. Now, amyloid is defined that "it stains orange-red by Congo red stain, and it is composed of accumulation of unbranched microfibril having a width of 8 to 15 nm causing green strongly brilliant birefringence when observed under a polarization microscope".

The amyloidosis causing an organ disorder by deposition of amyloid fibril includes various diseases such as inherited neurodegenerative diseases typically including familial amyloid polyneuropathy (FAP), Alzheimer's disease, Creutzfeldt-Jakob disease (mad cow disease), Huntington's disease in which amyloid-like substances accumulate in cells, and the like. Of them, FAP is systemic amyloidosis that causes amyloid deposition in whole body various organs such as peripheral nerve, autonomic nerve, kidney, skin and the like and inherits and permeates in the autosomal dominant inheritance mode, and a lot of cases thereof are confirmed in our country, particularly in Kyushu region and Chubu region. Since the first report of Portuguese FAP cases in 1952, similar cases have been reported from world countries. The amyloid fibril causing FAP is mainly constituted of a variant protein of transthyretin (TTR) as a serum protein.

Normal TTR has a steric conformation containing many β-sheet structures and is produced in liver, cerebral choroid plexus, retina, pancreatic α cell and the like, and particularly, it is said that 90% or more of this is produced in liver. Normal TTR forms a tetramer and undertakes a role as a transporter of vitamin A via thyroxine (T4), retinol-binding protein (RBP) and the like. As the variant of normal TTR, 100 or more variants have been reported so far, and it is clarified that the variants have low structural stability and tend to cause a conformational change from a tetramer to a monomer.

FAP which is dominant in our country is FAP caused by Val30Met type variant TTR as a causal protein. The main conditions of FAP are multiple neuritis accompanied by perceptual disorder symmetrically ascending from the lower limb end, and autonomic disorders (alternative diarrhea and constipation, postural hypotension, urinary disturbance and the like), and all of them are caused by nerve disorders due to amyloid. Thereafter, deposition of amyloid to heart, kidney and digestive tract becomes remarkable, causing malfunction of these organs. In general, it is a poor prognostic disease in which the disease develops in the late 20s to 30s, the patient becomes unable to walk within 10 years, and the patient dies of heart failure, renal failure and the like in the course of 10 years, and this is one of the refractory diseases that is also designated as specific diseases by Ministry of Health and Welfare.

The variant TTR protein that can cause FAP has lower structural stability as compared with the normal TTR protein, and it is said that β-sheet structures in the molecule assemble mutually to form insoluble amyloid fibril which is then deposits to tissue. For treating FAP, a liver transplantation treatment is conducted since about 90% or more of the variant TTR is produced in liver. In contrast, inhibition of dissociation of the tetramer TTR molecule is tried (Non-Patent documents 1 and 2) as the treatment method of FAP based on the amyloid fibril formation mechanism.

Further, the present inventors have suggested an amyloid fibril formation inhibitor containing as an active ingredient a cyclodextrin derivative modified with sugar, peptide or polyethylene glycol (Patent document 1). In this document, 6-O-α-(4-O-α-D-glucuronyl)-D-glycosyl-β-cyclodextrin and 6-O-α-maltosyl-β-cyclodextrin are disclosed as examples of the modified cyclodextrin derivative.

Furthermore, the present inventors have suggested an amyloid fibril formation inhibitor containing as an active ingredient a poly(amidoamine) dendrimer having an alkylenediamine as the core (Patent document 2).

Still further, the present inventors have reported inhibition of expression of TTR by a complex of a conjugate (GUG-β-CDE) of a polyamidoamine dendrimer (G2) and 6-O-α-(4-O-α-D-glucuronyl)-D-glycosyl-β-cyclodextrin with siRNA for TTR (siTTR), and in this document, utilization of GUG-β-CDE as a carrier for siRNA is suggested (Non-Patent document 3).

However, development of a therapeutic agent for refractory amyloidosis singly exhibiting a clinically sufficiently curative effect has not been succeeded yet. For this reason, drug combination therapy expecting an effect by combination use is envisaged, however, in this case, there is a possibility of generation of various problems such as generation of side effects due to drug interaction, and the like. Then, a drug showing a more efficacious curative effect has been desired.

### [Citation List]

### [Patent Document]

Patent document 1: JP-ANo. 2010-90054
Patent document 2: International Publication WO2011/002026

### [Non-Patent Document]

Non-Patent document 1: Yukio Ando, (2005), Med. Mol. Morphol., 38: pp. 142-154
Non-Patent document 2: Sekijima Y. "Recent progress in the understanding and treatment of transthyretin amyloidosis." (2014) J Clin. Pharm. Ther., 39: p225-33
Non-Patent document 3: "Effective utilization of dendrimer/glucuronylglucosyl-β-cyclodextrin conjugate as carrier for siRNA intending treatment of familial amyloid polyneuropathy", 28-th The Japan Society of Drug Delivery System Academic meeting lecture abstract, 2012

### Summary of the Invention

### Technical Problem

The present invention has an object of providing a more effective therapeutic agent for refractory amyloidosis.

More specifically, the present invention has an object of providing a novel substance capable of inhibiting formation of amyloid fibril that is highly safety and is more excellent in a TTR protein amyloid formation inhibiting effect as compared with conventional therapeutic agents. Furthermore, the present invention has an object of providing a medical drug comprising such a novel substance for prevention and/or treatment of amyloidosis, particularly, a medical drug comprising such a novel substance for prevention and/or treatment of familial amyloid polyneuropathy (FAP).

### Solution to Problem

The present inventors have intensively studied to solve the above-described problem and resultantly found that the therapeutic effects of GUG-β-CDE (fibril formation inhibition and fibril lysis) are enhanced remarkably, by forming a complex of the highly functional molecule dendrimer conjugate (GUG-β-CDE) created by the present inventors with a nucleic acid medical drug (shRNA or the like) intending inhibition of production of the causal protein by RNA interference, leading to completion of the present invention.

It is known that refractory amyloidosis progresses and develops by a change of steric conformation of the causal protein due to various triggers, and its development process progresses via three important steps, (1) increase of production of the amyloid causal protein or production of a variant protein, (2) amyloid fibrillation by a change of the steric conformation of the amyloid causal protein and (3) deposition of amyloid fibril to tissue. The GUG-β-CDE/nucleic acid medical drug complex provided by the present invention is also a novel multi-target type amyloidosis therapeutic agent that can exert a higher therapeutic effect than conventional ones, by inhibiting these three steps simultaneously.

The present invention includes the followings.
(1) An amyloid fibril suppressant comprising as an active ingredient a complex of a conjugate of cyclodextrin and polyamidoamine dendrimer with RNA causing RNA interference against mRNA of transthyretin.
(2) The amyloid fibril suppressant according to (1), wherein the cyclodextrin is glucuronylglucosyl-β-cyclodextrin (GUG-β-CyD).
(3) The amyloid fibril suppressant according to (1) or (2), wherein the RNA is shRNA or siRNA.
(4) The amyloid fibril suppressant according to (3), wherein the RNA is shRNA.
(5) The amyloid fibril suppressant according to any one of (2) to (4), wherein the conjugate is GUG-β-CDE (G2, DS 1.2), GUG-β-CDE (G2, DS 1.8), GUG-β-CDE (G2, DS 2.5) or GUG-β-CDE (G2, DS 4.5), as the conjugate (GUG-β-CDE) of glucuronylglucosyl-β-cyclodextrin and polyamidoamine dendrimer.
(6) The amyloid fibril suppressant according to any one of (2) to (5), wherein the charge ratio of GUG-β-CDE/RNA in the complex is 20 to 100.
(7) A pharmaceutical composition for prevention and/or treatment of amyloidosis comprising the amyloid fibril suppressant according to any one of (1) to (6).

### Advantageous Effect of the Invention

The amyloid fibril suppressant of the present invention can inhibit significantly both fibrillation of amyloid and deposition of amyloid fibril to tissue, since the suppressant has an excellent amyloid fibril lysis activity in addition to the excellent amyloid fibril formation inhibition activity. Further, the amyloid fibril suppressant of the present invention can also inhibit production of the amyloid causal protein, in addition to these actions. As described above, the pharmaceutical composition of the present invention comprising the amyloid fibril suppressant of the present invention is useful as a medical drug for prevention and/or treatment of amyloidosis, and particularly, useful for refractory amyloidosis, for example, familial amyloid polyneuropathy (FAP).

### Brief Explanation of Drawings

Fig. 1 is a view showing the effect of inhibition of formation of amyloid fibril by the GUG-β-CDE/shRNA complex (100 µM). In the figure, * represents p<0.05 as compared with the control and + represents p<0.05 as compared with GUG-β-CDE.
Fig. 2 is a view showing the effect of inhibition of formation of amyloid fibril at various concentrations (30 µM, 60 µM, 100 µM) of the GUG-β-CDE/shRNA complex. In the figure, * represents p<0.05 as compared with the control.
Fig. 3 is a view showing the effect of the amyloid fibril lysis action by the GUG-β-CDE/shRNA complex (100 µM).
Fig. 4 is a view showing the effect of inhibition of formation of fibril of Aβ amyloid as the causal protein of Alzheimer's disease, by the GUG-β-CDE/shRNA complex. In the figure, * represents p<0.05 as compared with the control and + represents p<0.05 as compared with GUG-β-CDE.
Fig. 5 is a view showing the effect of inhibition of formation of amyloid fibril using an amyloidosis mouse model, by the GUG-β-CDE/shRNA complex. The upper stage shows the result of Congo red stain, and the lower stage shows the result of detection of amyloid fibril.

### Description of Embodiments

Hereinafter, the present invention will be illustrated and described with reference to the exemplary embodiments, along with the preferred methods and materials which can be used in practice of the present invention. Unless otherwise specified in the sentences, any technical terms and scientific terms used in the present specification, have the same meaning as those generally understood by those of ordinary skill in the art to which the present invention belongs. Any materials and methods equivalent or similar to those described in the present specification can be used for practicing the present invention.

In the present specification, one having the action of inhibiting formation of amyloid fibril and the action of lysing the amyloid fibril formed in combination is called an amyloid fibril suppressant.

The amyloid fibril suppressant of the present invention contains as active ingredient a complex of a conjugate of cyclodextrin (CyD) and polyamidoamine dendrimer (CDE) with RNA causing RNA interference against mRNA of transthyretin (TTR) (hereinafter, referred to simply as "the complex of the present invention" in some cases).

The cyclodextrin constituting the cyclodextrin•polyamidoamine dendrimer conjugate of the present invention may be any of α, β or γ-cyclodextrin, and the α, β or γ-cyclodextrin can also be chemical modification type or non-modification type cyclodextrin. The general method for modifying cyclodextrin includes, for example, methylation, hydroxyalkylation such as hydroxyethylation, hydroxypropylation and the like, glucosylation, maltosylation, alkylation, acylation, acetylation, sulfation, sulfobutylation, carboxymethylation, carboxyethylation, amination, carboxylation, tosylation, dimethylacetylation and the like. The cyclodextrin is preferably β-cyclodextrin, further preferably glucuronylglucosyl-β-cyclodextrin (GUG-β-CyD). Thus, the preferable amyloid fibril suppressant of the present invention contains as an active ingredient a complex of a conjugate (GUG-β-CDE) of glucuronylglucosyl-β-cyclodextrin (GUG-β-CyD) and polyamidoamine dendrimer with RNA causing RNA interference against mRNA of transthyretin (TTR).

The polyamidoamine dendrimer constituting the cyclodextrin•polyamidoamine dendrimer conjugate of the present invention is a dendrimer having an alkylenediamine as the core, and the alkylenediamine as the core is not particularly restricted, and dendrimers having commonly used type of alkylenediamines are mentioned.

The complex of the present invention will be illustrated below using a conjugate (GUG-β-CDE) of glucuronylglucosyl-β-cyclodextrin (hereinafter, abbreviated as GUG-β-CyD in some cases) and a polyamidoamine dendrimer having an alkylenediamine as the core (hereinafter, referred to simply as "dendrimer" in some cases) as an example, but the present invention is not limited to this.

The conjugate (GUG-β-CDE) of glucuronylglucosyl-β-cyclodextrin (GUG-β-CyD) and a polyamidoamine dendrimer having an alkylenediamine as the core constituting the complex of the present invention can be obtained by bonding any GUG-P-CyD and any dendrimer according to an ordinary method. As GUG-P-CyD, 6-O-α-(4-O-α-D-glucuronyl)-D-glycosyl-β-cyclodextrin can be exemplified, but GUG-β-CyD is not limited to this. The dendrimer includes, but not limited to, for example, second to tenth generation, preferably second to sixth generation, more preferably second generation dendrimers.

The degree of substitution (DS) of the cyclodextrin in GUG-β-CDE used in the complex of the present invention is, for example, about 1.2 to about 4.5, preferably about 1.8, and such complexes can be used.

The conjugate of GUG-β-CyD and a dendrimer which is preferably used in the complex of the present invention is a conjugate of 6-O-α-(4-O-α-D-glucuronyl)-D-glycosyl-β-cyclodextrin and a second generation dendrimer, and is represented by the following structural formula.

### [Chemical formula 1]

Specific examples thereof include, but not limited to, GUG-β-CDE (G2, DS 1.2), GUG-β-CDE (G2, DS 1.8), GUG-β-CDE (G2, DS 2.5) and GUG-β-CDE (G2, DS 4.5).

The complex of the present invention is composed of the above-described conjugate (GUG-β-CDE) and RNA causing RNA interference against mRNA of transthyretin (TTR).

Any RNA may be used providing it causes RNA interference against mRNA of transthyretin (TTR), and examples thereof include shRNA and siRNA, and preferable is shRNA. The kind and the length of the RNA to be used are not particularly restricted providing the amyloid fibril formation inhibiting effect of GUG-β-CDE (at least one of amyloid fibril formation inhibiting effect and amyloid fibril lysis effect) can be enhanced by forming a complex with RNA.

In the present invention, the siRNA sequence which can be used in the present invention can be determined from the mRNA sequence of transthyretin (TTR). The sequence of mRNA transcript of human TTR can be found in NM_00371. Selection of the target site on the mRNA for siRNA used in the present invention can be conducted using known knowledge. For example, descriptions of documents such as Ui-Tei K., et al. Nucleic Acids Research (2004) 32 (3): 936-948 and the like can be referred to, but the selection condition is not limited to them. Further, for example, criteria such as (i) the GC content is about 30 to about 70%, preferably about 50%, (ii) all bases are equal, and G is not continuous, (iii) the base at the 5' end of an antisense strand is A or U, and the like, can be referred to, but the selection condition is not limited to them.

The siRNA used in the present invention should have a feature that when the siRNA is introduced into an animal cell (for example, human cell), RNA interference occurs, and production of transthyretin can be reduced. It is desirable not only to efficiently inhibit production of transthyretin as a target but also to have high selectivity not exerting an influence on expression of an unrelated gene (off-target effect) and not to develop undesirable toxicity and side effects of the oligo nucleic acid (siRNA) itself. Such a siRNA sequence can be determined by those skilled in the art based on known knowledge, and the siRNA itself can be obtained by those skilled in the art by fabricating and investigating according to ordinary methods (including, for example, fabricating actual siRNA, introducing it into a cell, and confirming transthyretin production inhibiting activity and toxicity to the cell). The absence of the off-target effect can be confirmed by the absence of a cross-reaction by previously utilizing Genechip or the like for the target siRNA, but the confirmation method is not limited to this.

Further, a sense strand sequence of the siRNA sequence which can be used in the present invention contains a sequence of 18 to 29 nucleotides, preferably 19 to 27 nucleotides, further preferably 19 to 25 nucleotides, more preferably 19 to 23 nucleotides continuing from mRNA of transthyretin or its alternatively spliced RNA. Using the sequence determined as described above, whether or not siRNA fabricated from the sequence generates the target effect of the present invention can be confirmed. Therefore, the fabricated siRNA is included in the present invention providing it generates the effect of the present invention, and also a pharmaceutical composition containing this and a method utilizing this are included in the present invention.

It is preferable that siRNA used in the present invention has an overhang at the end. The overhang of siRNA is located at the 5' or 3' end, preferably at the 3' end of RNA. The number of nucleotides constituting the overhang is about 1 to 5, preferably about 1 to 4, further preferably about 2 to 3, more preferably 2. It is preferable that the overhang is T or U, or G. As the overhang, siRNA having TT, UU or UG is preferable, but the overhang is not limited to this.

As the siRNA used in the present invention, for example, those currently under development as a therapeutic agent (for example, Revusiran) or those which are commercially available can be used, but the siRNA is not limited to them.

The siRNA used in the present invention may be a single siRNA, or a mixture of several siRNAs (so called, cocktail).

In the present invention, short hairpin RNA (shRNA) as the siRNA precursor can also be used. It is desirable that the short hairpin RNA (shRNA) as the siRNA precursor has an overhang composed of 2 to 4 Us at the 3' end of its antisense strand, and sense RNA and antisense RNA can get increased stability against decomposition by a nuclease because of the presence of the overhang.

The shRNA used in the present invention may be a single shRNA, or a mixture of several shRNAs (so called, cocktail).

The RNA used in the present invention can be synthesized by a chemically or gene recombinantly well-known method, and is easily synthesized chemically using a conventional RNA automatic synthesis apparatus in view of the number of nucleotides. It is also possible to fabricate the RNA by asking the siRNA-related custom synthesis company to synthesize it.

The charge ratio of GUG-β-CDE to RNA is not particularly restricted providing the amyloid fibril formation inhibiting effect of GUG-β-CDE (at least one of amyloid fibril formation inhibiting effect or amyloid fibril lysis effect) can be enhanced by forming a complex with RNA as show below, and the charge ratio is for example 20 to 200, preferably 50 to 150, particularly preferably 100.

Formation of a complex can be carried out by mixing both the compounds at the charge ratio within the above-described range.

The amyloid fibril suppressant of the present invention is a novel multi-target type amyloidosis therapeutic agent which can simultaneously inhibit three important steps, (1) increase of production of the amyloid causal protein or production of a variant protein, (2) amyloid fibrillation by a change of the steric conformation of the amyloid causal protein and (3) deposition of amyloid fibril to tissue, in the amyloidosis development process.

The amyloid fibril suppressant of the present invention in which GUG-β-CDE and a nucleic acid medical drug (shRNA and the like) expected to exhibit the RNA interference effect form a complex, created by the present inventors for the purpose of possibility of inhibiting the three steps (1) to (3) described above simultaneously, has a feature in which not only the effect of inhibiting production of the causal protein (step (1)) is added, but also the therapeutic effect (fibril formation inhibition•fibril lysis) of GUG-β-CDE itself is enhanced remarkably.

For example, the fibril formation inhibiting effect obtained by GUG-β-CDE is enhanced about 2 times or more by forming a complex with shRNA, as shown in the following example. The concentration of GUG-β-CDE showing the amyloid fibril formation inhibiting action can be halved approximately by forming a complex with shRNA. Further, also the fibril lysis action of GUG-β-CDE is significantly enhanced by forming a complex with shRNA.

As described above, the effect of enhancing fibril formation inhibition•fibril lysis by forming a complex of GUG-P-CDE with a nucleic acid medical drug (shRNA or the like) in the present invention has a feature of a multi-target type amyloidosis therapeutic agent by which a high therapeutic effect is exhibited even at low concentration, in addition to the effect capable of simultaneously inhibiting the above-described three steps. Further, safety is also improved since an efficacious effect can be shown at lower concentration.

The amyloid fibril suppressant of the present invention can effectively inhibit formation of amyloid fibril by the effect of enhancing the amyloid fibril formation inhibition-fibril lysis activity by formation of a complex with a nucleic acid in addition to inhibition of all the above-described steps (1) to (3), by comprising as an active ingredient a complex (GUG-β-CDE/nucleic acid) of GUG-β-CDE with a nucleic acid (siRNA, shRNA and the like) showing the RNA interference effect against mRNA of TRR.

The amyloid fibril suppressant of the present invention may contain any form of the complex of the present invention providing the inhibitor contains the complex of the present invention (GUG-β-CDE/nucleic acid). The ratio of the active ingredient contained in the amyloid fibril suppressant of the present invention (mass of active ingredient/mass of amyloid fibril formation inhibitor) is not particularly restricted providing amyloid fibril formation of variant TTR can be inhibited, and for example, it is preferably 80% to 100%.

The amyloid fibril suppressant of the present invention can be used in any form of solid or liquid providing the inhibitor contains the complex of the present invention (GUG-β-CDE/nucleic acid), and it is also possible to prepare a solid or liquid pharmaceutical composition by blending pharmaceutically acceptable carriers or additives to this.

Further, the present invention is a pharmaceutical composition for prevention and/or treatment of amyloidosis, comprising as an active ingredient the amyloid fibril suppressant of the present invention.

The pharmaceutical composition of the present invention can be applied widely to amyloidosis and amyloidosis-related diseases typically including familial amyloid polyneuropathy (FAP), senile amyloidosis and the like. Even for a patient already suffering from amyloidosis, an improvement of tissue having a disorder can be expected by applying the pharmaceutical composition of the present invention. Thus, according to the pharmaceutical composition of the present invention, not only prevention of amyloidosis and amyloidosis-related diseases and suppression of progress and worsening of the pathological conditions by these diseases, but also an improvement of the pathological conditions, that is, the therapeutic effect for these diseases, can be expected.

The disease targeted by the pharmaceutical composition of the present invention includes any of systemic amyloidosis and localized amyloidosis. Specific diseases of the systemic amyloidosis include, but not limited to, immunoglobulin-related amyloidosis (AL amyloidosis), AA amyloidosis, dialysis-related amyloidosis, familial amyloid polyneuropathy (FAP), senile systemic amyloidosis (SSA) and the like, and specific diseases of the localized amyloidosis include, but not limited to, Alzheimer's disease, cerebral amyloid angiopathy, prion disease and the like. The target of the present invention includes, particularly preferably, familial amyloid polyneuropathy (FAP), Alzheimer's disease, senile systemic amyloidosis (SSA) and AA amyloidosis.

Of patients who developed FAP, for example, for patients having mild condition, progress and worsening of the condition can be prevented by administering the pharmaceutical composition of the present invention, and also for patients having serious condition, the therapeutic effect can be expected in some cases. An individual having variant transthyretin is a potential patient having high possibility of developing FAP with aging, and development of FAP can be prevented by administering the pharmaceutical composition of the present invention.

As the pharmaceutical composition of the present invention, the amyloid fibril suppressant of the present invention may be used as it is, but in usual cases, it is desirable to prepare a form of a pharmaceutical composition containing one or more additives for preparation and the amyloid fibril suppressant of the present invention as an active ingredient.

In prevention and treatment of FAP, it is also desirable not to use only the medical drug of the present invention, but to use the medical drug of the present invention together with non-steroidal anti-inflammatory drugs for stabilizing transthyretin tetramer, for example, diflunisal and the like.

The pharmaceutical composition of the present invention is preferably a parenteral agent, and the pharmaceutical composition suitable for parenteral administration includes, for example, an injectable drug, a drop, an inhalant and the like. Additives for preparation used in production of the above-described pharmaceutical composition include, for example, an excipient such as lactose, oligosaccharide and the like, a disintegrating agent and a disintegration aid, a binder, a lubricant, a coating agent, a pigment, an antioxidative agent, a flavoring agent, a diluent, a base, a dissolving agent and a dissolving aid, a tonicity agent, a preserving agent, a pH regulator, a stabilizer, a propellant, an emulsifier, a suspending agent, a solvent, a filler, an extending agent, a buffering agent, a delivery vehicle, a carrier, a pharmaceutical adjuvant, a thickening agent and the like, and these can be selected appropriately by those skilled in the art depending on the form of the pharmaceutical composition, and two or more of them may be used in combination.

The more preferable form of the pharmaceutical composition of the present invention includes an injectable drug. Usually, the injectable drug does not substantially contain a non-aqueous solvent (or water-soluble organic solvent), and can be dissolved or diluted with a solvent in which the medium is substantially water. The injectable drug can be prepared by a method well known in the art. For example, the injectable drug can be prepared by dissolving in solvent media such as physiological saline, a buffering solution like PBS, sterilized water and the like, then, sterilizing the liquid by filtering through a filter or the like, then, filling it in an aseptic vessel (for example, ampule and the like). A conventional pharmaceutical carrier may be contained in this injectable drug, if necessary. Further, an administration method using a non-invasive catheter may be used. The carrier which can be used in the present invention includes neutral buffered physiological saline, physiological saline containing serum albumin, and the like.

A freeze-dried preparation (freeze-dried injectable drug) is also mentioned as a preferable form of the pharmaceutical composition of the present invention. Even such a freeze-dried preparation can be dissolved in at least one liquid or solvent selected from water for injection (distilled water for injection), infusion solutions including an electrolyte liquid (physiological saline or the like) and the like, nutrient infusion solutions and the like and an injection solution can be prepared easily, and as its vessel, a glass vessel and a plastic vessel can be used. The medical drug of the present invention can be contained in an amount of 0.01 part by weight or more, preferably 0.1 to 10 parts by weight with respect to 100 parts by weight of the content of the injectable drug.

The dosage, administration frequency and the like of the pharmaceutical composition of the present invention are not particularly restricted, and can be appropriately selected depending on conditions of a patient such as age, body weight, sex and the like, and the type and the degree of seriousness of a disease, the object of prevention or treatment, and the like. Usually, in the case of parenteral administration, the amount of an active ingredient is preferably 0.1 µg to 10 g, more preferably 10 µg to 1000 mg, further preferably 100 µg to 500 mg per day for an adult, and such a dosage may be administered in several divided doses a day. The frequency of administration of the medical drug of the present invention may be, for example, once a day to once every few months, and is not particularly restricted.

### EXAMPLES

The present invention will be illustrated below based on examples described below, but the present invention is not limited to the following examples.

### Example 1: Inhibition of formation of amyloid fibril (1)

### (1) Experimental material

Transthyretin (TTR) used in the present example was purified from serum obtained from a patient having Val30Met type mutation, according to guidelines approved by the Ethics Committee of Kumamoto University Graduate School of Life Science, which committee is defined by rules on bioethics.. Purity determination was conducted using unheated (non-reduced) SDS-PAGE.

6-O-α-(4-O-α-D-glucuronyl)-D-glycosyl-β-cyclodextrin (GUG-β-CyD) was purchased from Ensuiko Sugar Refining Co., Ltd., and polyamidoamine dendrimer (G2) was purchased from Sigma. GUG-β-CDE was prepared as described below. GUG-β-CyD (67.8 mg) was dissolved in dimethyl sulfoxide (DMSO), the condensing agent 4-(4,6-dimethoxy-1,3,5-triazin-2-yl)-4-methyl-morpholinium chloride (DMT-MM) (15.5 mg) was added, then, they were reacted at room temperature for 12 hours together with polyamidoamine dendrimer (G2) (0.5 mL), to obtain a conjugate (GUG-β-CDE). The resultant GUG-β-CDE was dialyzed for 48 hours (dialysis membrane: MWCO=3500), then, precipitated with ethanol, and purified by performing freeze dry.

A complex (GUG-β-CDE/shRNA) of GUG-β-CDE with RNA causing RNA interference against mRNA of TTR was prepared as described below, using the synthesized GUG-β-CDE and shRNA (TG308574, purchased from Origene). Into a 1.5 mL Eppendorf tube was added shRNA (5 µg) dissolved in tris-ethylenediaminetetraacetic acid (TE). Further, GUG-β-CDE dissolved in Hanks' Balanced Salt Solution (HBSS) was added, the mixture was vortexed for 10 seconds, then, incubated for 15 minutes at room temperature, to prepare a complex (GUG-β-CDE/shRNA).

### (2) Amyloid fibril formation method

Val30MetTTR was adjusted to a concentration of 20 µM with 1 mM glycine-hydrochloric acid buffer (pH 3.0) containing a phosphate buffering solution (PBS), and the liquid was incubated at 37°C, to form amyloid fibril.

### (3) Amyloid fibril measuring method

The prepared amyloid fibril was detected using thioflavin T as a fluorescent probe bonding selectively to the β-sheet polymerized. To 600 µL of a 500 µM thioflavin T solution dissolved in a 50 mM glycine solution, 3 µL of the incubated sample was added, then, mixed, to obtain a sample for measurement. Using a fluorescence spectrophotometer F-4500 manufactured by Hitachi, Ltd., the intensity of fluorescence of 489 nm exited with exciting light of 442 nm at 25°C was measured. The slit width was 10 nm at the excitation side and 20 nm at the fluorescence side.

### (4) Result of inhibition of amyloid fibril formation by GUG-P-CDE

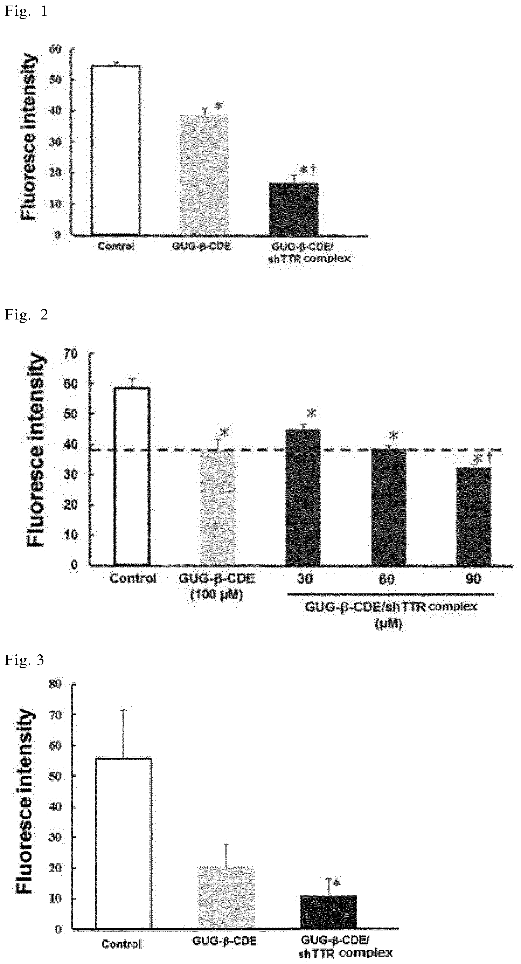

Using the above-described method, human serum-derived Val30MetTTR was amyloidized in presence or absence of 100 µM GUG-β-CDE/shRNA, amyloid fibril formation of TTR was quantitatively analyzed. As a control, GUG-P-CDE (100 µM) was used. The result is shown in Fig. 1. The fibril formation inhibiting effect (about 30%) obtained by GUG-β-CDE was enhanced about 2 times or more (about 70%) by forming a complex with shRNA.

### Example 2: Inhibition of formation of amyloid fibril (2)

According to the same manner as in Example 1, amyloid was formed in presence or absence of various concentrations (30 µM, 60 µM, 90 µM) of GUG-β-CDE/shRNA, and amyloid fibril formation of TTR was quantitatively analyzed. As a control, GUG-β-CDE (100 µM) was used. The result is shown in Fig. 2. The fibril formation inhibiting effect obtained by 100 µM GUG-P-CDE was confirmed at about half concentration (60 µM) by forming a complex with shRNA. That is, the concentration of GUG-β-CDE showing the amyloid fibril formation inhibiting action could be approximately halved by forming a complex with shRNA.

### Example 3: Amyloid fibril lysis

The amyloid fibril lysis action of the GUG-β-CDE/shRNA complex was confirmed. Specifically, the following process was conducted.

Amyloid fibril of human serum-derived Val30MetTTR was formed, then, the amyloid fibril was incubated for 6 hours in presence or absence of 100 µM GUG-β-CDE/shRNA. Thereafter, the amyloid fibril was quantitatively analyzed. As a control, GUG-β-CDE (100 µM) was used. The result is shown in Fig. 3. GUG-β-CDE (100 µM) shows the amyloid fibril lysis effect for TTR amyloid fibril once formed, and this effect was significantly enhanced by forming a complex with shRNA.

By forming a complex with shRNA, the remarkable amyloid fibril formation inhibiting effect could be obtained even at low concentration (100 µM) which is 1/500 of the concentration (50 mM) at which GUG-β-CyD exhibits the effect on amyloid fibril formation inhibition, and the effect on the lysis action was exhibited at low concentration (100 µM) which is 1/5 of the concentration (500 µM) at which the dendrimer itself exhibits the fibril lysis action (data not shown).

### Example 4: Inhibition of formation of Aβ 40 amyloid fibril

Amyloid β (Aβ 40) as the causal protein of Alzheimer's disease was made into amyloid fibril in the presence of GUG-β-CDE (100 µM) or GUG-β-CDE/shRNA (100 µM/0.1 µg) complex prepared by the method described in Example 1, and formation of Aβ 40 amyloid fibril was quantitatively analyzed using thioflavin T bonding selectively to the β sheet generated by amyloid fibrillation.

### (Aβ amyloid fibril formation method)

The concentration of Amyloid β-Protein (Human, 1-40, PEPTIDE INSTITUTE, INC.) was adjusted to 50 µM in a phosphate buffering solution (PBS), and the solution was incubated at 37°C for 72 hours, to form amyloid fibril.

### (Aβ amyloid fibril measurement method)

Aβ amyloid fibril was detected using thioflavin T. To 600 µL of a 500 µM thioflavin T solution dissolved in a 50 mM glycine solution, 6 µL of the sample was added, then, mixed, to give a sample for measurement, and the fluorescence intensity was measured (excitation at 445 nm, measurement at 490 nm).

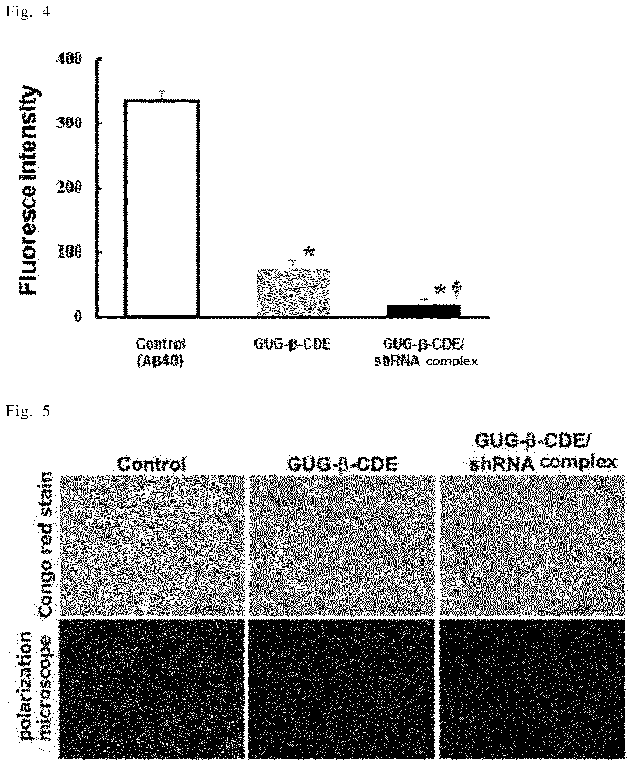

The result is shown in Fig. 4. GUG-β-CDE exhibited the remarkable inhibiting effect on Aβ 40 amyloid fibril formation, and the effect was significantly enhanced by forming a complex with shRNA (shRNA-control).

### Example 5: Amyloid fibril formation inhibition using amyloidosis mouse model

A mouse model of "AA amyloidosis (referred to also as secondary or reactive amyloidosis)" in which the metabolite amyloid A (AA) of serum amyloid A (SAA) deposits to tissue was used, and a change of tissue deposited amyloid (spleen tissue) by administering GUG-β-CDE or GUG-β-CDE/shRNA complex prepared by the method described in Example 1 was analyzed by Congo red stain and a polarization microscope.

### (AA amyloidosis model production method)

A C3H/HeN mouse (6-week old, male) was used, and 0.4 ml of 2% silver nitrate was administered subcutaneously to the back of the mouse and 3 ml of Amyloid-enhancing factor (AEF) (J Rheumatol. 33, 2260-70, 2006) was administered intraperitoneally, respectively, to produce an AA amyloidosis model. GUG-β-CDE (10 mg/kg) or GUG-β-CDE/shRNA (10 mg/kg) was intravenously injected at the tail of the mouse.

### (Amyloid fibril tissue deposition measurement method)

Five days after administration, spleen tissue revealing amyloid deposition in the AA amyloidosis model was collected, and amyloid fibril deposition was evaluated using Congo red stain.

A Congo red bulk powder (1 g) was dissolved in distilled water (100 ml), 45 g of NaCl was added while stirring, and the mixture was further stirred sufficiently, and 100 ml of ethanol was added and the mixture was cooled at 10°C, then, filtrated. The collected spleen tissue was stained for 1 hour with a Congo red stain liquid prepared by adding 5 g of phenol and 1 ml of acetic acid to 100 ml of this Congo red undiluted liquid, washed with water, then, the nuclear was stained with hematoxylin liquid. For detection of amyloid, this method was used, and amyloid was identified by apple green color under a polarization microscope.

The result is shown in Fig. 5. For the spleen tissue, amyloid fibril deposition was analyzed by Congo red stain and a polarization microscope, to confirm reduction of amyloid fibril in the GUG-P-CDE/shRNA administration group.

The foregoing merely illustrates objects and subjects of the present invention, and does not limit the accompanying Claims. Without departing from the accompanying Claims, various modifications and alterations to the described embodiments will be apparent to those skilled in the art in view of the teachings herein.

### Industrial Applicability

The disease causing organ disorders by deposition of amyloid fibril includes Alzheimer's disease, Creutzfeldt-Jakob disease (mad cow disease), Huntington's disease and other hereditary diseases, typically including FAP. For example, Alzheimer's disease occurs in about 10% of elderly people of 65 years or older, and it is a social problem in Japan becoming an increasingly aging society from now. Since deposition of amyloid fibril is ascribable to formation of insoluble fibril-formed aggregate of a protein, the complex of the present invention can also be applied to treatment of these various amyloidosis diseases.

## Claims

1. An amyloid fibril suppressant comprising as an active ingredient a complex of a conjugate of cyclodextrin and polyamidoamine dendrimer with RNA causing RNA interference against mRNA of transthyretin.

2. The amyloid fibril suppressant according to Claim 1, wherein the cyclodextrin is glucuronylglucosyl-β-cyclodextrin (GUG-β-CyD).

3. The amyloid fibril suppressant according to Claim 1 or 2, wherein the RNA is shRNA or siRNA.

4. The amyloid fibril suppressant according to Claim 3, wherein the RNA is shRNA.

5. The amyloid fibril suppressant according to any one of Claims 2 to 4, wherein the conjugate is GUG-β-CDE (G2, DS 1.2), GUG-β-CDE (G2, DS 1.8), GUG-β-CDE (G2, DS 2.5) or GUG-β-CDE (G2, DS 4.5), as the conjugate (GUG-β-CDE) of glucuronylglucosyl-β-cyclodextrin and polyamidoamine dendrimer.

6. The amyloid fibril suppressant according to any one of Claims 2 to 5, wherein the charge ratio of GUG-β-CDE/RNA in the complex is 20 to 100.

7. A pharmaceutical composition for prevention and/or treatment of amyloidosis comprising the amyloid fibril suppressant according to any one of Claims 1 to 6.

8. The pharmaceutical composition according to Claim 7, wherein the amyloidosis is familial amyloid polyneuropathy (FAP), Alzheimer's disease, senile systemic amyloidosis (SSA) or AA amyloidosis.
